(19) Europäisches Patentamt
European Patent Office
Office européen des brevets

(11) **EP 2 624 805 B1**

(12) **FASCICULE DE BREVET EUROPEEN**

(45) Date de publication et mention
de la délivrance du brevet:
**04.11.2015 Bulletin 2015/45**

(51) Int Cl.:
*A61K 8/06* *(2006.01)*     *A61K 8/34* *(2006.01)*
*A61K 8/44* *(2006.01)*     *A61K 8/60* *(2006.01)*
*A61Q 19/00* *(2006.01)*     *A61Q 19/08* *(2006.01)*

(21) Numéro de dépôt: **11771231.5**

(22) Date de dépôt: **20.09.2011**

(86) Numéro de dépôt international:
**PCT/FR2011/052158**

(87) Numéro de publication internationale:
**WO 2012/042148 (05.04.2012 Gazette 2012/14)**

(54) **NOUVELLES ÉMULSIONS COSMÉTIQUES EAU-DANS-HUILE COMPRENANT DES DÉRIVÉS N-ACYLÉ DE HAUT POINT DE FUSION ET PROCÉDÉS POUR LEURS PRÉPARATIONS**

NEUARTIGE KOSMETISCHE ÖL-IN-WASSER-EMULSIONEN MIT N-ACYLIERTEN DERIVATEN MIT HOHEM SCHMELZPUNKT UND VERFAHREN ZU IHRER HERSTELLUNG

NOVEL OIL-IN-WATER COSMETIC EMULSIONS INCLUDING N-ACYLATED DERIVATIVES HAVING A HIGH MELTING POINT, AND METHODS FOR PREPARING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.09.2010 FR 1057943**

(43) Date de publication de la demande:
**14.08.2013 Bulletin 2013/33**

(73) Titulaire: **Societe D Exploitation De Produits Pour Les
Industries Chimiques Seppic
75007 Paris (FR)**

(72) Inventeurs:
• **BARLERIN, Franck**
**F-81470 Cuq Toulza (FR)**
• **SHEN, Juanshu**
**F-81100 Castres (FR)**

(74) Mandataire: **Conan, Philippe Claude
L'Air Liquide SA
Direction de la Propriété Intellectuelle
75, quai d'Orsay
75321 Paris Cedex 07 (FR)**

(56) Documents cités:
**DE-A1-102008 012 988     FR-A1- 2 935 378
US-A1- 2009 298 783**

**Description**

La présente invention se rapporte aux formulations cosmétiques et/ou pharmaceutiques comprenant des dérivés N-acylés d'acides aminés.

Les dérivés N-acylés d'acides aminés, dans lesquels les radicaux acyles sont dérivés d'acides gras comportant de huit à vingt-quatre atomes de carbone, sont fréquemment utilisés, soit comme principes actifs, soit comme excipients dans des compositions cosmétiques ou pharmaceutiques. La demande de brevet européen publiée sous le numéro EP 1 449 517 divulgue par exemple l'utilisation de N-lauroyl aminoacides comme agent actif amincissant.

Les demandes internationales publiées sous les numéros WO2010023390 et WO2010026325 divulguent l'utilisation de dérivés N-acylés d'acides aminés parmi lesquels sont inclus le N-palmitoyl alanine, le N-palmitoyl glycine et le N-palmitoyl isoleucine, respectivement comme actif régulateur du profil génétique des fibroblastes réplicatifs sénescents du derme de la peau humaine dans des compositions anti-âge destinées à retarder l'apparition des rides et/ou à ralentir le vieillissement cellulaire de la peau du corps humain et, comme actif régulateur de la proportion de keratinocytes basaux de l'épiderme de la peau humaine exprimant la forme nucléaire de la survivine dans des compositions anti-âge destinées à maintenir la survivance de cellules souches au sein de l'épiderme de la peau humaine.

La demande de brevet français publiée sous le numéro 2 935 378 divulgue une composition cosmétique anti-âge comprenant la N-palmitoyl glycine et un mélange d'alcool arachidylique et d'alcool béhènylique.

Certains dérivés N-acylés ont des points de fusion supérieurs à 80°C. Il faut donc souvent les solubiliser à chaud dans un solvant approprié, avant d'être mis en oeuvre dans les formulations cosmétiques, ce qui exclut pour des raisons de sécurité et environnementales, des solvants performants mais à bas point d'ébullition comme par exemple les alcools légers comme l'éthanol, ou les glycols, qui sont connus pour générer des composés organiques volatils lors de leur mise en oeuvre à température élevée.

D'autres dérivés N-acylés d'acides aminés à caractère hydrophobe, comme par exemple l'alanine, la glycine, l'isoleucine, la leucine, la phényl alanine et la valine présentent également la caractéristique de montrer un caractère lipophile qui nécessite une introduction dans la phase grasse des compositions cosmétiques, éliminant *de facto* des solvants hydrosolubles conventionnels. Ils doivent donc être solubilisés dans la phase grasse de la composition cosmétique les comprenant.

De plus, de nombreuses émulsions cosmétiques de type huile-dans-eau comprennent des systèmes émulsionnants comprenant des tensioactifs qui génèrent *in situ* des cristaux liquides, comme par exemples les alkylpolyglycosides et les compositions comprenant des alcools gras et des alkylpolyglycosides. Or, lorsque de telles émulsions huile-dans-eau comprennent des dérivés N-acylés d'acides aminés hydrophobes, préalablement solubilisés dans la phase grasse, et un système tensioactif générateur de cristaux liquides, on observe la formation rapide de grumeaux, donnant alors un aspect non homogène à ladite émulsion huile-dans-eau, et/ou l'apparition lors de leur stockage, de cristaux solides comprenant ces acides aminés N-acylés.

Les inventeurs se sont donc attachés à développer une solution technique nouvelle qui permet de préparer des compositions cosmétiques se présentant sous la forme d'une émulsion huile-dans-eau, comprenant des dérivés N-acylés d'acides aminés dont le point de fusion est supérieur ou égal à 80°C et comprenant un système émulsionnant constitué d'au moins un tensioactif générateur de cristaux liquide, et dont l'aspect reste homogène dans le temps, en ne montrant pas l'apparition ni de grumeaux, ni de cristaux solides.

C'est pourquoi, selon un premier aspect, l'invention a pour objet une composition se présentant sous la forme d'une émulsion huile-dans-eau comprenant pour 100% de sa masse :

- (1) De 95% à 50% massique et plus particulièrement de 90% à 70% massique, <u>d'une phase aqueuse (P1)</u> cosmétiquement acceptable, et
- (2) De 5% à 50% massique et plus particulièrement de 10% à 30% massique <u>d'une phase grasse (P2),</u>

**caractérisée en ce que** ladite phase grasse (P2) comprend pour 100% de sa masse :

- a) De 0,2% à 60% massique, plus particulièrement de 2% à 20% massique, d'au moins un dérivé N-acylé d'acide aminé de formule (I) :

$$R_1\text{-C(=O)-NH-CH-(R'}_1)\text{-C(=O)-OH} \qquad (I)$$

dans laquelle $R_1$ représente un radical hydrocarboné aliphatique, saturé ou insaturé, linéaire ou ramifié, comportant de sept à dix-sept atomes de carbone, $R'_1$ représente un atome d'hydrogène, ou un radical hydrocarboné choisi parmi les radicaux méthyle, benzyle, isopropyle, 1-méthyl propyle ou isobutyle, et
- b) De 0,2% à 80% massique, plus particulièrement de 2% à 40% massique, d'un agent solubilisant comprenant pour 100% de sa masse :

- (i) De 5% à 100% massique d'au moins un composé de formule (II) :

$$R\text{-}OH \qquad (II)$$

dans laquelle R représente un radical alkyle ramifié $CH(C_nH_{2n+1})(C_mH_{2m+1})\text{-}CH_2\text{-}$ dans lequel m est un nombre entier pair compris entre 6 et 18, n est un nombre entier pair compris entre 4 et 18, et la somme n+m est supérieure à 10, et

- (ii) De 0% à 95% massique d'au moins un composé de formule (III) :

$$R'O\text{-}(X)p \qquad (III)$$

dans laquelle p représente un nombre décimal compris entre 1 et 5, X représente le reste de xylose et R', identique ou différent du radical R tel que défini ci-dessus, représente un radical alkyle ramifié $CH(C_nH_{2n+1})(C_mH_{2m+1})\text{-}CH_2\text{-}$ dans lequel m est un nombre entier pair compris entre 6 et 18, n est un nombre entier pair compris entre 4 et 18, et la somme n+m est supérieure à 10 ;

- c) De 5% à 99,4% massique, plus particulièrement de 5% à 94% massique, d'une ou plusieurs huile et/ou d'une ou plusieurs cires,
- d) De 0,2% à 80% massique, plus particulièrement de 2% à 40% massique, d'un système émulsionnant (A) comprenant pour 100% de sa masse :

  - (i) De 10% à 60% massique au moins un des éléments du groupe constitué par les alkylpolyglycosides de formule (IV) :

$$R_2\text{-}O\text{-}(G)_x\text{-}H \qquad (IV)$$

  dans laquelle le radical $R_2$ représente un radical aliphatique linéaire et saturé comportant de douze à vingt-deux atomes de carbone, G représente le reste d'un sucre réducteur choisi parmi le groupe constitué du glucose, du xylose et de l'arabinose, et x représente un nombre décimal supérieur ou égal à 1 et inférieur ou égal à 5, et
  - (ii) De 40% à 90% massique d'au moins un alcool gras de formule (V) :

$$R_3\text{-}OH \qquad (V)$$

dans laquelle le radical $R_3$, identique ou différent du radical $R_2$ tel que défini ci-dessus, représente un radical aliphatique linéaire saturé comportant de 12 à 22 atomes de carbone.

Le dérivé N-acylé d'acide aminé de formule (I) telle que définie ci-dessus peut se présenter sous forme acide libre ou sous forme partiellement ou totalement salifiée. Lorsque le dérivé N-acylé d'acide aminé de formule (I) est sous forme salifiée, il s'agit notamment de sels alcalins tels que les sels de sodium, de potassium ou de lithium, de sels alcalino-terreux tels que les sels de calcium, de magnésium ou de strontium ; de sel d'ammonium ou de sels d'amino-alcools comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium.

De façon générale, le taux de salification dudit dérivé N-acylé d'acide aminé de formule (I) dépendra en autre de son pKA et de la concentration en sels de la composition dans laquelle il est incorporé.

Dans le cadre de la présente invention, par dérivé N-acylé d'acide aminé de formule (I), on entend le dérivé N-acylé d'acide aminé de formule (I), sous forme libre ou partiellement ou totalement salifiée.

Le dérivé N-acylé de formule (I) est généralement obtenu par N-acylation d'un acide aminé sélectionné parmi le groupe comprenant la glycine, l'alanine, la leucine, l'isoleucine, la valine et la phényl alanine ou de leurs sels. La réaction d'acylation est connue de l'homme du métier. Elle est décrite par exemple dans la demande internationale publiée sous le numéro WO 98/09611. Elle est mise en oeuvre indifféremment sur un acide aminé ou sur un mélange d'acides aminés. L'agent d'acylation consiste généralement en un dérivé activé d'un acide carboxylique de formule :

$$R_1\text{-}C(=O)\text{-}OH,$$

dans laquelle $R_1$ est tel que défini précédemment, tel qu'un anhydride symétrique de cet acide, l'ester méthylique de cet acide, ou un halogénure d'acide comme le chlorure d'acide ou le bromure d'acide.

Il peut aussi consister en un mélange de dérivés activés d'acides carboxyliques issus d'huiles ou graisses naturelles d'origine animales ou végétales telles que les huiles de coprah, de palmiste, de palme, de soja, de colza, de maïs, le suif de boeuf, l'huile spermaceti ou l'huile de hareng.

Dans le cadre de la présente invention on utilise de préférences les mélanges d'acides gras issus de l'huile de coprah, de l'huile de palmiste, de l'huile de spermaceti ou de l'huile de noix de coco qui, contiennent une fraction majoritaire en acide dodécanoïque :

| | huile de coprah | huile de palmiste | huile de spermaceti | huile de noix de coco |
|---|---|---|---|---|
| acide octanoïque ou caprylique ($C_8H_{16}O_2$) | 6% à 9% | 3% à 10% | - | 2% à 10% |
| acide décanoïque ou caprique ($C_{10}H_{20}O_2$) | 6% à 10% | 3% à 14% | 1% à 3% | 3% à 9% |
| acide dodécanoïque ou laurique ($C_{12}H_{24}O_2$) | 44% à 51% | 37% à 52% | 14% à 38% | 45% à 56% |
| acide tétradécanoïque ou myristique ($C_{14}H_{28}O_2$) | 13% à 18% | 7% à 17% | 12% à 14% | 15% à 23% |
| acide hexadécanoïque ou palmitique ($C_{16}H_{32}O_2$) | 8% à 10% | 2% à 9% | 8% à 10% | 7% à 14% |
| acide octadécanoïque ou stéarique ($C_{18}H_{36}O_2$) | 1% à 3% | 1% à 3% | 1% à 3% | 1% à 7% |
| acide octadécénoïque ou oléique ($C_{18}H_{34}O_2$) | 5,5% à 7,5% | 11 % à 23% | 15% à 18% | 3% à 11% |
| acide eicosénoïque ou gadolique ($C_{20}H_{38}O_2$) | - | - | 5% à 8% | - |
| acide octadécadiénoïque ou linoléique ($C_{18}H_{32}O_2$) | < 2,5% | 1% à 3% | - | $\leq$ 3% |
| autres acides | < 0,4% | < 0,6% | 26% à 34% | < 3% |

Selon un aspect particulier, l'invention a pour objet une composition telle que définie précédemment, caractérisée en ce que dans la formule (I), le radical $R_1$-C(=O)-représente un radical choisi parmi les radicaux octanoyle, décanoyle, dodécanoyle, tétradécanoyle, hexadécanoyle, octadécanoyle ou octadécènoyle.

Selon un autre aspect particulier, l'invention a pour objet une composition telle que définie précédemment, caractérisée en ce que dans la formule (I), le radical $R'_1$ représente un atome d'hydrogène ou un radical hydrocarboné choisi parmi les radicaux méthyle, isopropyle, 1-méthyl propyle ou isobutyle ; et plus particulièrement un atome d'hydrogène ou un radical hydrocarboné choisi parmi les radicaux méthyle, 1-méthyl propyle ou isobutyle.

Selon ce dernier aspect particulier, le composé de formule (I) est choisi parmi le N-palmitoyl alanine, le N-palmitoyl glycine, le N-palmitoyl leucine, le N-palmitoyl isoleucine ou le N-cocoyl alanine.

Par radical cocoyl, on entend dans l'exposé de la présente invention un radical issu du mélange d'acides gras provenant de l'huile de noix de coco dont la composition est décrite dans le tableau précédent.

Selon un autre aspect particulier, l'invention a pour objet la composition telle que définie précédemment, pour laquelle dans les formules (II) et (III), les radicaux R et R', identiques ou différents, représentent un radical choisi parmi les radicaux 2-hexyl décyle, 2-hexyl dodécyle, 2-octyl décyle, 2-octyl dodécyle, 2-décyl tétradécyle.

Les composés de formule (II), constitutifs de l'agent solubilisant dans la phase grasse (P2) de l'émulsion cosmétique objet de la présente invention, sont des alcools ramifiés obtenus selon le procédé Guerbet consistant en une réaction chimique permettant de convertir un alcool aliphatique linéaire en un alcool dimère β-alkylé avec la perte d'une mole d'eau. Ces composés de formule (II) sont commercialisés sous le nom de marque ISOFOL™ par la société SASOL et sous le nom de marque EUTANOL™ par la société COGNIS. Dans la définition du composés de formule (III) telle que définie précédemment, p est un nombre décimal qui représente le degré moyen de polymérisation du reste de xylose. Lorsque p est un nombre entier, (X)p est le reste polymérique de rang p du reste de xylose. Lorsque p est un nombre décimal, la formule (III) représente un mélange de composés :

$$a_1 R'\text{-}O\text{-}(X)_1\text{-}H + a_2 R'\text{-}O\text{-}(X)_2\text{-}H + a_3 R'\text{-}O\text{-}(X)_3\text{-}H + ... + a_q R'\text{-}O\text{-}(X)_q\text{-}H$$

avec q représentant un nombre entier compris entre 1 et 5 et dans les proportions molaires $a_1$, $a_2$, $a_3$,... $a_q$ telles que :

$$\sum_{q=5}^{q=1} a_q = 1 \; ; \; a_1 > 0$$

Selon un autre aspect particulier de la présente invention, dans la définition des composés de formules (I), p est compris entre 1,05 et 5, et plus particulièrement entre 1,05 et 2.

La structure oligomérique $(X)_p$ peut se présenter sous toute forme d'isomérie, qu'il s'agisse d'isomérie optique, d'isomérie géométrique ou d'isomérie de position ; elle peut aussi représenter des mélanges d'isomères. Dans la formule (III) telle que définie ci-dessus, le radical R'-O- est lié à X par le carbone anomérique du reste saccharidique, de manière à former une fonction acétal.

Les composés de formule (III) sont préparés par réaction du xylose avec un excès d'alcool de formule R'-OH, pour lequel R' représente un radical alkyle ramifié $CH(C_nH_{2n+1})(C_mH_{2m+1})-CH_2-$ dans lequel m est un nombre entier pair compris entre 6 et 18, n est un nombre entier pair compris entre 4 et 18, et la somme n+m est supérieure à 10. Cette réaction est généralement mise en oeuvre dans un réacteur en présence d'un système catalytique acide, en maîtrisant le rapport stoechiométrique entre les deux réactants, et avec agitation mécanique dans des conditions de température et de vide partiel prédéterminées, par exemple à une température comprise entre 70°C et 130°C et sous un vide partiel compris entre 300 mbar ($3.10^4$ Pa) et 20 mbar ($2.10^3$ Pa). Par système catalytique acide, on désigne les acides forts comme l'acide sulfurique, l'acide chlorhydrique, l'acide phosphorique, l'acide nitrique, l'acide hypophosphoreux, l'acide méthanesulfonique, l'acide (para-toluène) sulfonique, l'acide (trifluorométhane) sulfonique, ou les résines échangeuses d'ions. L'alcool de formule R'-OH n'ayant pas réagi est éliminé selon des méthodes connues de l'homme du métier comme par exemple, la distillation, la distillation dur film à couche mince, la distillation moléculaire ou l'extraction par solvants. Un tel procédé de préparation peut être complété, si nécessaire ou si désiré, par des opérations de neutralisation, de filtration et de décoloration.

Dans la composition se présentant sous la forme d'une émulsion cosmétique huile-dans-eau objet de la présente invention et telle que définie précédemment, parmi les huiles constitutives de la phase grasse (P2), on peut citer :

- les huiles minérales telles que l'huile de paraffine, l'huile de vaseline, les isoparaffines ou les huiles blanches minérales ;
- les huiles d'origine animale, telles que le squalène ou le squalane ;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, l'huile de sésame, l'huile de reine des prés, l'huile de Macadamia kiwi, l'huile de bourrache, l'huile de pépins de cassis, l'huile de café, l'huile de pistache, l'huile de noyau de pêche, l'huile de pépin de framboise, l'huile de pépin de fraise, l'huile de melon, l'huile de pépin de myrtille, l'huile d'argan, l'extrait huileux de prune, l'huile de grenade, l'huile de papaye, l'huile de lait de coco, les huiles issues de fleurs ou de légumes ;
- les huiles végétales éthoxylées ;
- les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylyl éther, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, les isoalcanes de synthèse, le polydécène hydrogéné ou le polyisobutène hydrogéné, commercialisé en France par la société Ets B. Rossow et Cie sous le nom PARLEAM - POLYSYNLANE™, cité dans : Michel and Irene Ash ; Thesaurus of Chemical Products, Chemical Publishing Co, Inc. 1986 Volume I, page 211 (ISBN 0 7131 3603 0), les huiles perfluorées ; et
- les huiles de silicone comme les diméthylpolysiloxanes, les méthylphényl-polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Dans la composition se présentant sous la forme d'une émulsion cosmétique huile-dans-eau objet de la présente invention et telle que définie précédemment, parmi les cires constitutives de la phase grasse (P2), on peut citer la cire d'abeille, la cire de carnauba, la cire de candelilla, la cire d'ouricoury, la cire du Japon, la cire de fibre de liège, la cire de canne à sucre, la cire de jojoba, la cire de fleurs de cassis, la cire de fleurs de narcisse, la cire de fleurs d'oranger, la cire d'orange, la cire de riz, les cires de paraffines, les cires de lignite, les cires microcristallines, la cire de lanoline ; l'ozokérite ; la cire de polyéthylène, les cires de silicone ; les cires végétales ; les alcools gras et les acides gras solides à température ambiante ; les glycérides solides à température ambiante.

Dans la définition des composés de formule (IV) présents dans le système émulsionnant (A) compris dans la composition se présentant sous la forme d'une émulsion cosmétique huile-dans-eau objet de la présente invention et telle que définie précédemment, x est un nombre décimal qui représente le degré moyen de polymérisation du reste G.

Lorsque x est un nombre entier, $(G)_x$ est le reste polymérique de rang x du reste G. Lorsque x est un nombre décimal, la formule (IV) représente un mélange de composés :

$$a_1\ R_2\text{-O-}(G)_1\text{-H} + a_2\ R_2\text{-O-}(G)_2\text{-H} + a_3\ R_2\text{-O-}(G)_3\text{-H} + ... + a_q\ R_2\text{-O-}(G)_q\text{-H}$$

avec q représentant un nombre entier compris entre 1 et 5 et dans les proportions molaires $a_1$, $a_2$, $a_3$,... $a_q$ telles que :

$$\sum_{q=5}^{q=1} a_q = 1 \ ; \ a_1 > 0$$

Selon un autre aspect particulier de la présente invention, dans la définition des composés de formules (IV), x est compris entre 1,05 et 5, et plus particulièrement entre 1,05 et 2.

Par reste d'un sucre réducteur, on désigne pour G dans la définition des composés de formules (IV) un reste de dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique $(G)_x$ peut se présenter sous toute forme d'isomérie, qu'il s'agisse d'isomérie optique, d'isomérie géométrique ou d'isomérie de position ; elle peut aussi représenter des mélanges d'isomères.

Dans la formule (IV) telle que définie ci-dessus, le radical $R_2$ est lié à G par le carbone anomérique du reste saccharide, de manière à former une fonction acétal.

Selon un aspect particulier de la présente invention, par reste d'un sucre réducteur, on désigne dans la définition du composé de formule (IV) telle que définie ci-dessus, le reste du glucose.

Selon un aspect plus particulier de la présente invention, dans la définition du composé de formule (IV) compris dans le système émulsionnant (A) contenu dans la composition se présentant sous la forme d'une émulsion cosmétique huile-dans-eau objet de la présente invention, le radical $R_2$ est choisi parmi les radicaux dodécyle, tétradécyle, hexadécyle, octadécyle et 12-hydroxy stéaryle.

Les composés de formule (IV) sont préparés par réaction du sucre réducteur G-OH avec un excès d'alcool de formule $R_2$-OH, pour lequel $R_2$ représente un radical aliphatique linéaire et saturé comportant de 12 à 22 atomes de carbone. Cette réaction est généralement mise en oeuvre dans un réacteur en présence d'un système catalytique acide, en maîtrisant le rapport stoechiométrique entre les deux réactants, et avec agitation mécanique dans des conditions de température et de vide partiel prédéterminées, par exemple à une température comprise entre 70°C et 130°C et sous un vide partiel compris entre 300 mbar ($3.10^4$ Pa) et 20 mbar ($2.10^3$ Pa). Par système catalytique acide, on désigne les acides forts comme l'acide sulfurique, l'acide chlorhydrique, l'acide phosphorique, l'acide nitrique, l'acide hypophosphoreux, l'acide méthanesulfonique, l'acide (para-toluène) sulfonique, l'acide (trifluorométhane) sulfonique, ou les résines échangeuses d'ions. L'alcool de formule $R_2$-OH n'ayant pas réagi est éliminé selon des méthodes connues de l'homme du métier comme par exemple, la distillation, la distillation dur film à couche mince, la distillation moléculaire ou l'extraction par solvants. Un tel procédé de préparation peut être complété, si nécessaire ou si désiré, par des opérations de neutralisation, de filtration et de décoloration.

Selon un aspect plus particulier de la présente invention, dans la définition des alcools gras de formule (V) compris dans le système émulsionnant (A) contenu dans la composition se présentant sous la forme d'une émulsion cosmétique huile-dans-eau objet de la présente invention, le radical $R_3$ est choisi parmi les radicaux dodécyle, tétradécyle, hexadécyle, octadécyle et 12-hydroxy stéaryle.

Lorsque le système émulsionnant (A) comprend à la fois un composé de formule (IV) telle que définie ci-dessus ou un mélange de composés de formule (IV) et un composé de formule (V) telle que définie ci-dessus ou un mélange de composés de formule (V), avec les radicaux $R_2$ et $R_3$ identiques, une première voie de préparation_du système émulsionnant (A) consiste à mélanger dans les proportions massiques souhaitées, le composé de formule (IV) ou un mélange de composés de formule (IV), avec le composé de formule (V), ou un mélange de composés de formule (V).

Une seconde voie de préparation d'un système émulsionnant tel que décrit précédemment consiste à mettre en oeuvre un procédé de réaction du sucre réducteur G-OH avec un excès d'alcool de formule $R_2$-OH, généralement mise en oeuvre dans un réacteur en présence d'un système catalytique acide, tel que décrit précédemment pour la préparation du composé de formule (IV), en maîtrisant le rapport stoechiométrique entre les deux réactants, et dans des conditions de température et de vide partiel prédéterminées, telles que décrites précédemment pour la préparation du composé de formule (IV). La maitrise du rapport stoechiométrique entre les deux réactants permet de s'affranchir de l'étape d'élimination de l'alcool de formule $R_2$-OH telle que décrite précédemment pour la préparation du composé de formule (IV) et un tel procédé de préparation peut être complété, si nécessaire ou si désiré, par des opérations de neutralisation, de filtration et de décoloration.

Lorsque le système émulsionnant (A) comprend à la fois un composé de formule (IV) telle que définie ci-dessus ou un mélange de composés de formule (IV) et un composé de formule (V) telle que définie ci-dessus ou un mélange de composés de formule (V), avec les radicaux $R_2$ et $R_3$ différents, une voie de préparation_du système émulsionnant (A) consiste à mélanger dans les proportions massiques souhaitées, le composé de formule (IV) ou un mélange de composés de formule (IV), avec le composé de formule (V), ou un mélange de composés de formule (V).

L'expression "cosmétiquement acceptable" utilisée dans la définition de la composition objet de la présente invention, signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état. Un milieu cosmétiquement acceptable de ces compositions objet de l'invention peut contenir classiquement de l'eau, un ou plusieurs solvants organique cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, l'hexylène glycol, le diéthylène glycol, le xylitol, l'érythritol, le sorbitol, ou les alcools hydrosolubles.

Selon un autre aspect particulier de la présente invention, la composition telle que définie précédemment comprend en outre, pour 100% de sa masse, de 0,1% à 5% massique, plus particulièrement de 0,1% à 2% massique, d'un polymère épaississant et/ou gélifiant.

Parmi les polymères épaississants et/ou gélifiants que l'on peut associer à la composition se présentant sous la forme d'une émulsion cosmétique huile-dans-eau telle que définie précédemment et objet de la présente invention, on peut citer :

- les polymères de type polyélectrolytes comme par exemple les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800, SIMULGEL™ A, SIMULGEL™ EPG, SIMULGEL™ INS, SIMULGEL™ FL, SIMULGEL SMS 88, SEPIGEL™ 501, SEPIGEL™ 502, SEPIPLUS™ 250, SEPIPLUS™ 265, SEPIPLUS™ 400, SEPIPLUS S, SEPINOV™ EMT 10, SEPIMAX™ZEN, CARBOPOL™, ULTREZ™ 10, ACULYN™, PEMULEN™ TR1, PEMULEN™ TR2, LUVIGEL™ EM, SALCARE™ SC91, SALCARE™ SC92, SALCARE™ SC95, SALCARE™ SC96, FLOCARE™ ET100, FLOCARE™ ET58, HISPAGEL™, NOVEMER™ EC1, ARISTOFLEX™ AVC, ARISTOFLEX™ HBM, RAPITHIX™ A60, RAPITHIX™ A100, COSMEDIA SP et STABILEZE™ 06 ;
- les hydrocolloïdes d'origine végétale ou biosynthétique, par exemple la gomme de xanthane, la gomme de karaya, les carraghénates, les alginates, les galactomannanes;
- les silicates ; la cellulose et ses dérivés ; l'amidon et ses dérivés hydrophiles ; les polyuréthanes.

Les compositions se présentant sous la forme d'une émulsion huile-dans-eau telles que définies précédemment et objets de la présente invention peuvent se présenter sous la forme de crèmes, de laits, de gels crèmes, de lotions fluides, de lotions fluides vaporisables.

De façon générale, ces compositions se présentant sous la forme d'émulsions huile-dans-eau telles que définies précédemment et objets de la présente invention sont administrées aux humains ou aux animaux par voie topique. L'expression "par voie topique" utilisée signifie que ladite composition est mise en oeuvre par application sur la peau, les cheveux, le cuir chevelu ou les muqueuses, qu'il s'agisse d'une application directe dans le cas d'une composition cosmétique, dermocosmétique, ou d'une application indirecte par exemple dans le cas d'un produit d'hygiène corporelle sous forme de lingette en textile ou en papier ou de produits sanitaires destinés à être en contact. avec la peau ou les muqueuses.

De façon générale ces compositions se présentant sous la forme d'émulsions huile-dans-eau telles que définies précédemment et objets de la présente invention, comportent également des excipients et ou des principes actifs habituellement mis en oeuvre dans le domaine des formulations à usage topique, en particulier cosmétiques, dermocosmétiques, pharmaceutiques ou dermo-pharmaceutiques, comme les tensioactifs épaississants et/ou gélifiants, les stabilisants, les composés filmogènes, les agents hydrotropes, les agents plastifiants, les agents émulsionnants et co-émulsionnants, les agents opacifiants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les repellents pour les insectes.

Comme exemples de tensioactifs épaississants et/ou gélifiants éventuellement présents dans les compositions se présentant sous la forme d'émulsions huile-dans-eau objets de la présente invention, on peut citer :

- les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120
- Les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.
- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples d'émulsionnants éventuellement présents dans les compositions se présentant sous la forme d'émulsions huile-dans-eau objets de la présente invention, on peut citer : - les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120.

- Les esters gras alkoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141.
- Les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.
- Les acides gras, les acides gras éthoxylés, les esters d'acide gras et de sorbitol, les esters d'acide gras et de mannitol, les esters d'acides gras éthoxylés, les polysorbates, les esters de glycérol tel que le stéarate de glycérol et la composition comprenant du stéarate de glycérol et du PEG-100 stéarate commercialisée par la société SEPPIC sous l'appellation SIMULSOL™165, les esters de polyglycérols tels que les polyhydroxystéarates de polyglycérols, les esters de polyglycol tels que les polyhydroxystéarates de polyglycols, les esters de polyols les alcools gras éthoxylés, les esters de sucrose, les alcools gras sulfatés, les alcools gras phosphatés.

Comme exemples d'agents opacifiants et/ou nacrants éventuellement présents dans les compositions se présentant sous la forme d'émulsions huile dans eau objets de la présente invention, on peut citer les palmitates ou les stéarates ou les hydroxystéarates de sodium ou de magnésium, les monostéarates ou distéarates d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.

Comme exemples d'agents de texture éventuellement présents dans les compositions se présentant sous la forme d'émulsions huile dans eau objets de la présente invention, on peut citer des dérivés N-acylés d'acides aminés, comme la lauroyl lysine commercialisée sous l'appellation AMINOHOPE™LL par la société AJINOMOTO, les polyméthacrylates de méthyle commercialisés sous l'appellation MICROPEARL™ par la société SEPPIC, le nylon-12.

Comme exemples de filtres solaires éventuellement présents dans les compositions se présentant sous la forme d'émulsions huile dans eau objets de la présente invention, on peut citer tous ceux figurant dans la directive cosmétique 76/768/CEE modifiée annexe VII.

Comme exemples de principe actif éventuellement présents dans les compositions se présentant sous la forme d'émulsions huile dans eau objets de la présente invention, on peut citer : les composés ayant une action éclaircissante ou dépigmentante tels que par exemple l'arbutine, l'acide kojique, l'hydroquinone, l'acide ellagique, la vitamine C ou les dérivés de la Vitamine C comme par exemple le magnésium ascorbyl phosphate, les extraits de polyphénols, les extraits de raisin, les extraits de pin, les extraits de vin, les extraits d'olives, les extraits de marc, les extraits de jus de pomme , les dérivés d'acides aminés comme par exemple l'undécélénoyl phenylalanine commercialisé sous l'appellation SEPIWHITE MSH, les peptides ; les hydrolysâts totaux de protéines ; les hydrolysâts partiels de protéines ; les polyols (comme par exemple, la glycérine ou le butylène glycol) ; l'urée ; l'hydroxy éthyl urée ; l'acide pyrrolidonecarboxylique ou les dérivés de cet acide ; l'acide glycyrrhétinique ; l'alpha-bisabolol ; les sucres ou les dérivés des sucres ; les polysaccharides ou leurs dérivés ; les hydroxyacides par exemple l'acide lactique ; les vitamines ou les dérivés de vitamines comme par exemple le Rétinol, la vitamine E et ses dérivés ; les sels minéraux ; les enzymes ; les co-enzymes comme par exemple le Coenzyme Q10 ; les hormones ou "hormone like" comme par exemple le PHYTO-AGE™ ; les extraits de soja comme par exemple la Raffermine™ ; les extraits de blé comme par exemple la Tensine™ ou la Gliadine™ ; les extraits végétaux tels que les extraits riches en tanins, les extraits riches en isoflavones ou les extraits riches en terpènes ; les extraits d'algues d'eau douce ou d'algues marines ; les extraits de plantes marines ; les cires essentielles ; les extraits bactériens ; les lipides en général et plus particulièrement les lipides tels que les céramides ou les phospholipides ; les actifs ayant une activité antimicrobienne ou une action purifiante vis à vis des peaux grasses ; les actifs ayant une propriété énergisante ou stimulante comme le SEPITONIC™ M3 ou le Physiogényl™ ; le panthénol et ses dérivés comme le SEPICAP™ MP; les actifs anti-âge comme le SEPIVINOL™, le SEPIVITAL™ , l'EXTRAMEL™ C, le MANOLIVA™; les actifs hydratants comme l'AQUAXYL™ ; les actifs " anti-photo vieillissement " ; les actifs présentant une action de tenseur ou de lissage immédiat sur la peau comme par exemple le SESAFLASH™ ; les actifs protecteurs de l'intégrité de la jonction dermo-épidermique comme le PHYTO-AGE™; les actifs augmentant la synthèse des composants de la matrice extracellulaire comme le SEPITONIC™ M3, l'AQUAXYL™ ; les actifs ayant une activité amincissante, raffermissante ou drainante comme par exemple la caféine, les dérivés de la caféine, la théophylline, l'Adénosyl Mono Phosphate cyclique (AMPc), l'ADIPOLESS™ , le thé vert, la sauge, le ginko biloba, le lierre, le marron d'inde, le bambou, le ruscus, le petit houx, la centella asiatica, la bruyère, l'ulmaine, le fucus, le romarin, la saule , les extraits de panais, les extraits de potentille ; des actifs créant une sensation de « chauffe » sur la peau tels que les activateurs de la microcirculation cutanée comme exemple les nicotinates ; les actifs créant une sensation de « fraîcheur » sur la peau comme par exemple le menthol et ses dérivés ; les actifs présentant une action vis à vis des cellules souches ; les actifs présentant une action sur l'épiderme, le derme, l'hypoderme et les annexes cutanées (poils, ongles, glandes sébacées, pores ...) ; les actifs présentant une action vis à vis de la flore cutanée ; les agents de bronzage et/ou de brunissement artificiel de la peau comme par exemple les composés mono- ou polycarbonylés tels que la dihydroxyacétone, l'isatinne, l'alloxane, la ninhydrine, le glycéraldéhyde, l'aldéhyde mésotartrique, la glutaraldéhyde, l'érythrulose.

L'invention a aussi pour objet un procédé de préparation de la composition telle que définie précédemment, caractérisé en ce qu'il comprend :

- Au moins une étape a) de préparation de ladite phase grasse (P2) par mélange pour 100% massique de la dite phase (P2) :

  1) De 0,2% à 60% massique, plus particulièrement de 2% à 20% massique, d'au moins un dérivé N-acylé d'acide aminé de formule (I), avec
  2) De 0,2% à 80% massique, plus particulièrement de 2% à 40% massique, dudit agent solubilisant ;
  3) De 5% à 99,4% d'une ou de plusieurs huiles et/ou d'une ou de plusieurs cires et et avec
  4) De 0,2% à 80% massique, plus particulièrement de 2% à 40% massique, dudit système émulsionnant (A) ; et
  5) Optionnellement un polymère épaississant et/ou gélifiant, en la quantité nécessaire pour obtenir dans ladite compostion de 0,1% à 5% massique, plus particulièrement de 0,1% à 2% massique dudit un polymère épaississant et/ou gélifiant,

- Au moins un étape b) d'émulsification de 5% à 50% massique de ladite phase grasse P2 dans de 95% à 50% massique de ladite phase aqueuse P1 .

Selon un aspect particulier, l'étape a) de préparation de la phase grasse (P2) est réalisée à une température comprise entre 70° C et 90°C, plus particulièrement entre 80°C et 90°C.

Dans le procédé objet de l'invention, l'étape a) de préparation de la phase grasse (P2) telle que décrite ci-dessus peut être réalisée par le moyen de tout dispositif de mélange connu de l'homme du métier, comme par exemple par le moyen d'un dispositif d'agitation équipé d'un mobile de type « ancre », à des vitesses d'agitations comprises entre 80 tours/minute et 1000 tours/minute, et comme par exemple par le moyen d'un dispositif d'agitation de type rotor-stator à des vitesses d'agitation comprises entre 1000 tours/minute et 8000 tours /minute.

Dans le procédé tel que défini précédemment, l'étape b) d'émulsification est avantageusement réalisée à une température comprise entre 70° C et 90°C, plus particulièrement entre 80°C et 90°C, et par le moyen d'un dispositif d'agitation de type rotor-stator à des vitesses d'agitation comprises entre 1000 tours/minute et 8000 tours /minute.

L'invention a aussi pour objet l'utilisation de la composition telle que définie précédemment, en tant que composition topique cosmétique par son application sur la peau ou les muqueuses du corps humain

Les exemples suivants illustrent l'invention, sans toutefois la limiter.

## Exemple 1 : Préparation d'émulsions huile-dans-eau comprenant un dérivé N-acylé de formule (I) selon l'invention et selon l'état de la technique.

On prépare une série d'émulsions huile-dans-eau (Emulsions E1 à E3), dont les compositions sont indiquées dans le tableau 1 ci-dessous, en mettant en oeuvre dans le procédé suivant :

**Etape a) :** Le système émulsionnant est ajouté sur les huiles préalablement introduites dans un réacteur porté à une température de 85°C et le mélange obtenu est homogénéisé pendant 30 minutes par l'intermédiaire d'un agitateur muni d'un mobile de type ancre, à une vitesse de 80 tours/minute. Le dérivé N-acylé de formule (I) est ensuite ajouté à ce mélange avec l'agent solubilisant à une température de 85°C et la phase grasse ainsi obtenue est homogénéisé pendant 30 minutes par l'intermédiaire d'un agitateur muni d'un mobile de type ancre, à une vitesse de 80 tours/minute.

**Etape b) :** Le polymère épaississant est introduit sur le mélange préparé à l'issue de l'étape a) maintenu à une température de 85°C. Le mélange résultant est homogénéisé pendant 15 minutes par l'intermédiaire d'un agitateur muni d'un mobile de type ancre, à une vitesse de 80 tours/minute.

**Etape c) :** Les ingrédients de la phase aqueuse sont ajoutés sur le mélange préparé à l'issue de l'étape b), et le mélange résultant est mainenu à une température de 85°C, puis soumis à une agitation par l'intermédiaire d'un émulseur de type rotor-stator, commercialisé par la société SILVERSON, pendant une durée de 4 minutes à une vitesse de 4000 tours/minute. Le mélange résultant de l'étape c) est ensuite refroidi à température ambiante.

*Tableau 1 :* Composition des émulsions E1 à E3.

|  | Emulsion E1 (selon l'invention) | Emulsion E2 comparative | Emulsion E3 comparative |
|---|---|---|---|
| **Phase grasse** | | | |
| *Système émulsionnant :* | | | |
| - Montanov™ L[1] | 4% | 4% | 4% |
| *Huiles :* | | | |
| - Cera Alba | 3% | 3% | 3% |
| - Butyrospermum Parkii Shea Butter | 2,3% | 2,3% | 2,3% |
| - Huile de jojoba | 2% | 2% | 2% |
| - Glyceryl caprylate | 0,5% | 0,5% | 0,5% |
| - Lanol™ 99[2] | 3,5% | 3,5% | 3,5% |
| - alcool cétéarylique | 0,5% | 0,5% | 0,5% |
| *Agent solubilisant* | | | |
| - Octyl-2 dodécanol-1 | 7% | 0% | 0% |
| - Caprylic/Capric triglycerides | 0% | 7% | 0% |
| - Mono propylene glycol | 0% | 0% | 7% |
| - Palmitoyl Isoleucine[5] | 1% | 1% | 1% |
| ***Polymère épaississant*** | | | |
| - Sepinov™ EMT10[3] | 0,7% | 0,7% | 0,7% |
| ***Phase aqueuse*** | | | |
| - Eau | Qsp 100% | Qsp 100% | Qsp 100% |

(suite)

| Phase aqueuse | | | |
|---|---|---|---|
| - Sepicide™ HB[(4)] | 1% | 1% | 1% |

(1) MONTANOV™ L (nom INCI C14-22 ALCOHOL/C12-20 ALKYL GLUCOSIDE), est une composition auto-émulsionnable commercialisée par la société SEPPIC consistant en un mélange d'alcools tétradécylique, hexadécylique, octadécylique, béhénique et arachidylique avec des dodécylpolyglucosides, des tétradécylpolyglucosides, des hexadécylpolyglucosides, des octadécylpolyglucosides, des arachidylpolyglucosides et des béhénylpolyglucosides.
(2) LANOL™ 99 est de l'isononanoate d'isononyle commercialisé par la société SEPPIC.
(3) SEPINOV™ EMT 10 est un agent épaississant comprenant un copolymère réticulé d'acryloyldiméthyl taurate de sodium et d'hydroxyéthyl acrylate se présentant sous la forme d'une poudre et commercialisé par la société SEPPIC.
(4) SEPICIDE™ HB, qui est un mélange de phénoxyéthanol, de méthylparaben, d'éthylparaben, de propylparaben et de butylparaben, est un agent conservateur commercialisé par la société SEPPIC.
(5) Le palmitoyl Isoleucine se caractérise par un point de fusion de 92°C.

La mesure du point de fusion est réalisée à l'aide d'un appareil automatique, de marque METTLER munie d'une cellule de type FP81 et liée à une unité de commande FP90, commercialisé par la société Mettler, permettant de saisir des modifications thermo-optiques de la substance étudiée : un échantillon de la substance à analyser est introduit dans un tube capillaire en verre, lequel est placé dans l'appareil de mesure et chauffé avec une vitesse de chauffe définie. Une cellule photo-électrique de l'appareil enregistre de façon continue l'intensité de la lumière transmise, soit de la transparence, de l'échantillon. Le point de fusion se caracétise alors par la température à laquelle la transparence de l'échantillon est détectére par la cellule photoélectrique.

La moitié de la quantité de chaque émulsion ainsi préparée est ensuite conservée dans une enceinte climatique isolée et régulée à une température de 20°C pendant un mois. L'autre moitié de la quantité de chaque émulsion ainsi préparées est conservée dans une enceinte climatique isolée et régulée à une température de 45°C pendant un mois.

A l'issue de cette période d'un mois, l'aspect de chaque émulsion préparée, stockée à 20°C et à 45°C, est observé et la viscosité des émulsions formées sont également mesurées respectivement à 20°C et à 45°C. En ce qui concerne les émulsions sotckées à 45°C pendant un mois, une quantité desdites émulsions est mise en stabilité dans une enceinte climatique isolée et régulée à 20°C pendant 24 heures et la viscosité est ensuite mesurée.

Les résultats obtenus et observés sont consignés dans le tableau 2 ci-dessous.

*Tableau 2* : *caractérisation des émulsions E1 à E3.*

| | Emulsion E1 (selon l'invention) | Emulsion E2 comparative | Emulsion E3 comparative |
|---|---|---|---|
| Aspect après un mois à 20°C | Homogène : absence de grumeaux et de cristaux | Hétérogène : présence de grumeaux et de cristaux | Hétérogène : présence de grumeaux et de cristaux |
| Viscosité après un mois à 20°C (viscosimètre Brookfiel RVT, Mobile 7, Vitesse 5 tours/min) | 110 000 mPa.s | 136 000 mPa.s | 120 000 mPa.s |
| Viscosité après un mois à 45°C (viscosimètre Brookfiel LVT, Mobile 4, Vitesse 6 tours/min) | 49 000 mPa.s | 47 000 mPa.s | 45 000 mPa.s |
| Viscosité après un mois à 45°C et mise en stabilité consécutive à 20°C (viscosimètre Brookfiel LVT, Mobile 4, Vitesse 6 tours/min) | 95 000 mPa.s | 90 000 mPa.s | 90 000 mPa.s |

Les résultats compris dans le tableau 2 font apparaitre que l'émulsion huile-dans-eau (E1) comprenant l'octyl-2 dodécanol-1 dans la phase grasse est stable et homogène après un stockage d'une durée d'un mois à 20°C alors que les émulsions (E2) et (E3) présente un aspect hétérogène se caractérisant par la présence de grumeaux, de grains et de cristaux pendant la même période de stockage dans les mêmes conditions.

De plus, lorsque l'émulsion (E1) selon l'invention a été stockée pendant un mois à une température de 45°C puis remise consécutivement en stabilité à 20°C, la différence de viscosité par rapport à un stockage à 20°C pendant un mois est constituée par une perte de 13,6% de la valeur de la viscosité à 20°C, alors que cette perte s'élève à 33,8% pour l'émulsion (E2) et à 25% pour l'émulsion (E3) traduisant ainsi une meilleure stabilisation de l'émulsion selon l'invention.

**Formulations**

Dans les formules suivantes, les pourcentages sont exprimés en pourcentage massique pour 100% de la masse de la formulation.

**Exemple 2 : Crème visage fermeté « volufirming »**

| | |
|---|---|
| Phase A : | |
| LANOL™ P : | 1,5% |
| MONTANOV™ 202 : | 2,0% |
| MONTANOV™ 82 | 1,0% |
| Squalane : | 7,0% |
| Polyisobutène | 10,0% |
| FLUIDANOV 20X | 1,0% |
| N-palmitoyl Isoleucine | 1,0% |
| Phase B : | |
| Eau | qsp 100% |
| Chlorphénésine | 0,3% |
| Triéthanolamine | qsp pH 6,0 |
| Glycérol | 5,0% |
| Phase C : | |
| Gomme de xanthane | 0,15% |
| Phase D : | |
| SEPIPLUS™ 400 | 0,8% |
| Phase E : | |
| SEPICIDE™ LD | 0,7% |
| Parfum/fragrance | 0,1% |

Procédé de préparation :

Introduire les ingrédients de la phase A séparément dans un réacteur à une température de 85°C, et mélanger pendant 30 minutes à 85°C avec un agitateur mécanique muni d'une pâle de type « ancre ».

Ajouter la phase D sur la phase A à une température de 85°C sous agitation et homogéniser toujours à 85°C.

Préparer séparément la phase B par ajout des différents ingrédients dans l'eau. Porter la phase B à 85°C et ajouter la phase C à la phase B sous agitation à une température de 85°C.

Ajouter la phase (A + D) sur la phase (B + C) à une température de 85°C et émulsionner ce mélange à l'aide d'un agitateur muni d'un rotor-stator à une vitesse de 4000 tours/minutes pendant 4 minutes. Refroidir le mélange obtenu sous agitation avec un mobile de type « ancre » à une vitesse de 100 tours/minutes en refroidissant pendant 30 minutes de façon à atteindre une température de 40°C. Ajouter alors la phase E.

**Exemple 3 : Crème buste fermeté «potion push-up »**

| Phase A : | |
|---|---|
| Eau | qsp 100% |
| Glycérol | 3,0% |
| Phase B : | |
| N-palmitoyl Isoleucine | 1,0% |
| Huile minérale | 6,0% |
| Butyrospermum Parkii (Shea Butter) | 1,5% |
| FLUIDANOV™ 20X | 2,0% |
| Néopentyl Glycol Diéthylhexanoate | 5,0% |
| MONTANOV™202 : | 2,0% |
| Phase C: | |
| SIMULGEL™INS 100 | 1,0% |
| Phase D: | |
| Phenoxyéthanol and Methylparaben and | |
| Propylparaben and Methylisothiazolinone | 0,5% |
| Parfum/fragrance | 0,3% |

Procédé de préparation :

Introduire les ingrédients de la phase B séparément dans un réacteur à une température de 85°C, et mélanger pendant 30 minutes à 85°C avec un agitateur mécanique muni d'une pâle de type « ancre ». Préparer séparément la phase A par ajout des différents ingrédients dans l'eau. Porter la phase A à 85°C. Ajouter la phase C sur la phase B sous agitation à une température de 85°C.

Ajouter la phase (B + C) sur la phase A à une température de 85°C et émulsionner ce mélange à l'aide d'un agitateur muni d'un rotor-stator à une vitesse de 4000 tours/minutes pendant 4 minutes.

Refroidir le mélange obtenu sous agitation avec un mobile de type « ancre » à une vitesse de 100 tours/minutes en refroidissant pendant 30 minutes de façon à atteindre une température de 40°C. Ajouter alors la phase D.

**Exemple 4 : Crème jeunesse fraicheur de teint**

| Phase A : | |
|---|---|
| LANOL™ P : | 1,5% |
| MONTANOV™202 : | 2,0% |
| MONTANOV™82 | 1,0% |
| Caprylic/Capric Triglycéride | 7,0% |
| Polyisobutène | 6,0% |
| Octyl-2 dodécanol-1 | 5,0% |
| Diméticone | 2,0% |
| N-palmitoyl Glycine | 1,0% |
| Phase B : | |
| Eau | qsp 100% |
| Triéthanolamine | qsp pH 5,0 |
| Glycérol | 5,0% |
| Phase C : | |
| Gomme de xanthane | 0,15% |
| Phase D : | |
| SEPIPLUS™400 | 0,7% |
| Phase E : | |
| Phenoxyéthanol and EthylHexylglycérine | 1,0% |
| Parfum/fragrance | 0,1% |

Procédé de préparation :

Introduire les ingrédients de la phase A séparément dans un réacteur à une température de 85°C, et mélanger pendant 30 minutes à 85°C avec un agitateur mécanique muni d'une pâle de type « ancre ».

Ajouter la phase D sur la phase A à une température de 85°C sous agitation et homogéniser toujours à 85°C.

Préparer séparément la phase B par ajout des différents ingrédients dans l'eau. Porter la phase B à 85°C et ajouter la phase C à la phase B sous agitation à une température de 85°C.

Ajouter la phase (A + D) sur la phase (B + C) à une température de 85°C et émulsionner ce mélange à l'aide d'un agitateur muni d'un rotor-stator à une vitesse de 4000 tours/minutes pendant 4 minutes. Refroidir le mélange obtenu sous agitation avec un mobile de type « ancre » à une vitesse de 100 tours/minutes en refroidissant pendant 30 minutes de façon à atteindre une température de 40°C. Ajouter alors la phase E.

Les définitions des produits commerciaux utilisés dans les exemples, sont les suivantes :

LANOL™ P (nom INCI : Glycol Palmitate) est un ester utilisé comme phase huileuse dans la préparation de composition cosmétique et distribué par la société SEPPIC.

MONTANOV™ 202 (nom INCI : Arachidyl Alcohol and Behenyl Alcohol and Arachidyl Glucoside) est une composition auto-émulsionnante commercialisée par la société SEPPIC.

MONTANOV™ 82 (nom INCI : Cetearyl Alcohol and Coco Glucoside) est une composition auto-émulsionnante commercialisée par la société SEPPIC.

FLUIDANOV 20X (nom INCI : Octyldodecanol and Octyldodecyl Xyloside) est une composition commercialisée par la société SEPPIC.

SEPIPLUS™ 400 (nom INCI : Polyacrylate-13 and Polyisobutene and Polysorbate 20) est une composition épaississante polymérique se présentant sous la forme d'une émulsion inverse (latex inverse) concentrée, commercialisée par la société SEPPIC. SEPICIDE™ LD est un agent conservateur contenant du phénoxyéthanol, commercialisé par la société SEPPIC.

SIMULGEL™ INS 100 (nom INCI : Hydroxyethyl Acrylate/Sodium Acryloydimethyl Taurate Copolymer and Isohexadecane and Polysorbate 60) est une composition épaississante polymérique se présentant sous la forme d'une émulsion inverse (latex inverse), commercialisée par la société SEPPIC.

## Revendications

**1.** Composition se présentant sous la forme d'une émulsion huile-dans-eau comprenant pour 100% de sa masse :

- (1) De 95% à 50% massique et plus particulièrement de 90% à 70% massique, <u>d'une phase aqueuse (P1)</u> cosmétiquement acceptable, et
- (2) De 5% à 50% massique et plus particulièrement de 10% à 30% massique <u>d'une phase grasse (P2),</u>

**caractérisée en ce que** ladite phase grasse (P2) comprend pour 100% de sa masse :

- a) De 0,2% à 60% massique, plus particulièrement de 2% à 20% massique, d'au moins un dérivé N-acylé d'acide aminé de formule (I) :

$$R_1\text{-C(=O)-NH-CH-(R'}_1\text{)-C(=O)-OH} \qquad (I)$$

dans laquelle $R_1$ représente un radical hydrocarboné aliphatique, saturé ou insaturé, linéaire ou ramifié, comportant de sept à dix-sept atomes de carbone, $R'_1$ représente un atome d'hydrogène, ou un radical hydrocarboné choisi parmi les radicaux méthyle, benzyle, isopropyle, 1-méthyl propyle ou isobutyle, et
- b) De 0,2% à 80% massique, plus particulièrement de 2% à 40% massique, d'un agent solubilisant comprenant pour 100% de sa masse :

- (i) De 5% à 100% massique d'au moins un composé de formule (II) :

$$R\text{-OH} \qquad (II)$$

dans laquelle R représente un radical alkyle ramifié $CH(C_nH_{2n+1})(C_mH_{2m+1})$-$CH_2$- dans lequel m est un nombre entier pair compris entre 6 et 18, n est un nombre entier pair compris entre 4 et 18, et la somme n+m est supérieure à 10, et

- (ii) De 0% à 95% massique d'au moins un composé de formule (III) :

$$R'O-(X)p \qquad (III)$$

dans laquelle p représente un nombre décimal compris entre 1 et 5, X représente le reste de xylose et R', identique ou différent du radical R tel que défini ci-dessus, représente un radical alkyle ramifié $CH(C_nH_{2n+1})(C_mH_{2m+1})$-$CH_2$- dans lequel m est un nombre entier pair compris entre 6 et 18, n est un nombre entier pair compris entre 4 et 18, et la somme n+m est supérieure à 10 ;

- c) De 5% à 99,4% massique, plus particulièrement de 5% à 94% massique, d'une ou plusieurs huile et/ou d'une ou plusieurs cires,
- d) De 0,2% à 80% massique, plus particulièrement de 2% à 40% massique, d'un système émulsionnant (A) comprenant pour 100% de sa masse :

- (i) De 10% à 60% massique d'au moins un des éléments du groupe constitué par les alkylpolyglycosides de formule (IV) :

$$R_2-O-(G)_x-H \qquad (IV)$$

dans laquelle le radical $R_2$ représente un radical aliphatique linéaire et saturé comportant de douze à vingt-deux atomes de carbone, G représente le reste d'un sucre réducteur choisi parmi le groupe constitué du glucose, du xylose et de l'arabinose, et x représente un nombre décimal supérieur ou égal à 1 et inférieur ou égal à 5, et
- (ii) De 40% à 90% massique d'au moins un alcool gras de formule (V) :

$$R_3-OH \qquad (V)$$

dans laquelle le radical $R_3$, identique ou différent du radical $R_2$ tel que défini ci-dessus, représente un radical aliphatique linéaire saturé comportant de 12 à 22 atomes de carbone.

2. Composition telle que définie à la revendication 1, **caractérisée en ce que** dans la formule (I), le radical $R_1$-C(=O)- représente un radical choisi parmi les radicaux octanoyle, décanoyle, dodécanoyle, tétradécanoyle, hexadécanoyle, octadécanoyle ou octadécènoyle.

3. Composition telle que définie à l'une des revendications 1 ou 2, **caractérisée en ce que** dans la formule (I), le radical $R'_1$ représente un atome d'hydrogène ou un radical hydrocarboné choisi parmi les radicaux méthyle, isopropyle, 1-méthyl propyle ou isobutyle.

4. Composition telle que définie à la revendication , **caractérisée en ce que** dans la formule (I), le radical $R'_1$ représente un atome d'hydrogène ou un radical hydrocarboné choisi parmi les radicaux méthyle, 1-méthyl propyle ou isobutyle.

5. Composition telle que définie à la revendication 4, dans laquelle le dérivé N-acylé d'acide aminé de formule (I) est le N-palmitoyl alanine, le N-palmitoyl glycine, le N-palmitoyl leucine, le N-palmitoyl isoleucine ou le N-cocoyl alanine.

6. Composition telle que définie à l'une des revendications 1 à 5, pour laquelle dans les formules (II) et (III), les radicaux R et R', identiques ou différents, représentent un radical choisi parmi les radicaux 2-hexyl décyle, 2-hexyl dodécyle, 2-octyl décyle, 2-octyl dodécyle, 2-décyl tétradécyle.

7. Composition telle que définie à l'une ou quelconque des revendications 1 à 6, **caractérisée en ce qu'**elle comprend en outre pour 100% de sa masse de 0,1% à 5% massique, plus particulièrement de 0,1% à 2% massique, d'un polymère épaississant et/ou gélifiant.

8. Procédé de préparation de la composition telle que définie à l'une des revendication 1 à 7, **caractérisé en ce qu'**il comprend :

- <u>Au moins une étape a)</u> de préparation de ladite phase grasse (P2) par mélange pour 100% massique de ladite phase (P2) :

a) De 0,2% à 60% massique, plus particulièrement de 2% à 20% massique, d'au moins un dérivé N-acylé d'acide aminé de formule (I), avec

b) De 0,2% à 80% massique, plus particulièrement de 2% à 40% massique, dudit agent solubilisant ;

c) De 5% à 99,4% d'une ou de plusieurs huiles et/ou d'une ou de plusieurs cires et et avec

d) De 0,2% à 80% massique, plus particulièrement de 2% à 40% massique, dudit système émulsionnant (A) ; et

e) Optionnellement un polymère épaississant et/ou gélifiant, en la quantité nécessaire pour obtenir dans ladite compostion de 0,1% à 5% massique, plus particulièrement de 0,1% à 2% massique dudit un polymère épaississant et/ou gélifiant,

- Au moins un étape b) d'émulsification de 5% à 50% massique de ladite phase grasse P2 dans de 95% à 50% massique de ladite phase aqueuse P1.

9. Utilisation de la composition telle que définie à l'une des revendications 1 à 7, en tant que composition topique cosmétique par son application sur la peau ou les muqueuses du corps humain.

## Patentansprüche

1. Zusammensetzung, die in Form einer Öl-in-Wasser-Emulsion vorliegt und die pro 100 % ihrer Masse Folgendes enthält:

(1) 95 Massen-% bis 50 Massen-%, und insbesondere 90 Massen-% bis 70 Massen-%, einer kosmetisch verträglichen wässrigen Phase (P1) und

(2) 5 Massen-% bis 50 Massen-%, und insbesondere 10 Massen-% bis 30 Massen-%, einer Fettphase (P2),

**dadurch gekennzeichnet, dass** die Fettphase (P2) pro 100 % ihrer Masse Folgendes enthält:

a) 0,2 Massen-% bis 60 Massen-%, insbesondere 2 Massen-% bis 20 Massen-%, wenigstens eines N-Acyl-aminosäurederivats der Formel (I):

$$R_1\text{-C(=O)-NH-CH-}(R'_1)\text{-C(=O)-OH} \qquad (I)$$

worin $R_1$ ein geradkettiges oder verzweigtes, gesättigtes oder ungesättigtes aliphatisches Kohlenwasserstoffradikal mit sieben bis siebzehn Kohlenstoffatomen darstellt, $R'_1$ ein Wasserstoffatom oder ein Kohlenwasserstoffradikal darstellt, das ausgewählt ist aus den Methyl-, Benzyl-, Isopropyl-, 1-Methylpropyl- oder Isobutyl-Radikalen, und

b) 0,2 Massen-% bis 80 Massen-%, insbesondere 2 Massen-% bis 40 Massen-%, eines Lösungsvermittlers, der pro 100 % seiner Masse Folgendes enthält:

(i) 5 Massen-% bis 100 Massen-% wenigstens einer Verbindung der Formel (II):

$$R\text{-OH} \qquad (II)$$

worin R ein verzweigtes Alkylradikal $CH(C_nH_{2n+1})(C_mH_{2m+1})\text{-}CH_2\text{-}$ darstellt, worin m eine gerade ganze Zahl zwischen 6 und 18 ist, n eine gerade ganze Zahl zwischen 4 und 18 ist und die Summe aus n + m größer als 10 ist, und

(ii) 0 Massen-% bis 95 Massen-% wenigstens einer Verbindung der Formel (III):

$$R'O\text{-}(X)p \qquad (III)$$

worin p eine Dezimalzahl zwischen 1 und 5 darstellt, X den Xyloserest darstellt und R', das mit dem Radikal R, wie es oben definiert ist, identisch oder von ihm verschieden ist, ein verzweigtes Alkylradikal $CH(C_nH_{2n+1})(C_mH_{2m+1})\text{-}CH_2\text{-}$ darstellt, worin m eine gerade ganze Zahl zwischen 6 und 18 ist, n eine gerade ganze Zahl zwischen 4 und 18 ist und die Summe aus n + m größer als 10 ist;

c) 5 Massen-% bis 99,4 Massen-%, insbesondere 5 Massen-% bis 94 Massen-%, eines oder mehrerer Öle und/oder eines oder mehrerer Wachse, b) 0,2 Massen-% bis 80 Massen-%, insbesondere 2 Massen-% bis 40 Massen-%, eines Emulgatorsystems (A), das pro 100 % seiner Masse Folgendes enthält:

(i) 10 Massen-% bis 60 Massen-% wenigstens eines der Elemente der Gruppe bestehend aus den Alkyl-polyglycosiden der Formel (IV):

$$R_2\text{-O-}(G)_x\text{-H} \qquad (IV)$$

worin das Radikal $R_2$ ein geradkettiges und gesättigtes aliphatisches Radikal mit zwölf bis zweiundzwanzig Kohlenstoffatomen darstellt, G den Rest eines reduzierenden Zuckers darstellt, der aus der Gruppe bestehend aus Glukose, Xylose und Arabinose ausgewählt ist, und x eine Dezimalzahl darstellt, die größer oder gleich 1 und kleiner oder gleich 5 ist, und

(ii) 40 Massen-% bis 90 Massen-% wenigstens eines Fettalkohols der Formel (V):

$$R_3\text{-OH} \quad (V)$$

worin das Radikal $R_3$, das mit dem oben definierten Radikal $R_2$ identisch oder von ihm verschieden ist, ein gesättigtes geradkettiges aliphatisches Radikal mit 12 bis 22 Kohlenstoffatomen darstellt.

2. Zusammensetzung nach Anspruch 1, **dadurch gekennzeichnet, dass** in der Formel (I) das Radikal $R_1$-C(=O)- ein Radikal darstellt, das ausgewählt ist aus den Octanoyl-, Decanoyl-, Dodecanoy-, Tetradecanoyl-, Hexadecanoyl-, Octadecanoyl- oder Octadecenoyl-Radikalen.

3. Zusammensetzung nach einem der Ansprüche 1 oder 2, **dadurch gekennzeichnet, dass** in der Formel (I) das Radikal $R'_1$ ein Wasserstoffatom oder ein Kohlenwasserstoffradikal darstellt, das ausgewählt ist aus den Methyl-, Isopropyl-, 1-Methylpropyl- oder Isobutyl-Radikalen.

4. Zusammensetzung nach Anspruch [sic], **dadurch gekennzeichnet, dass** in der Formel (I) das Radikal $R'_1$ ein Wasserstoffatom oder ein Kohlenwasserstoffradikal darstellt, das ausgewählt ist aus den Methyl-, 1-Methylpropyl- oder Isobutyl-Radikalen.

5. Zusammensetzung nach Anspruch 4, in der das N-Acylaminosäurederivat der Formel (I) N-Palmitoyl-Alanin, N-Palmitoyl-Glycin, N-Palmitoyl-Leucin, N-Palmitoyl-Isoleucin oder N-Cocoyl-Alanin ist.

6. Zusammensetzung nach einem der Ansprüche 1 bis 5, bei der in den Formeln (II) und (III) die Radikale R und R', die identisch oder verschieden sind, ein Radikal darstellen, das ausgewählt ist aus den 2-hexyl-decyl-, 2-hexyldodecyl-, 2-octyl-decyl-, 2-octyl-dodecyl-, 2-decyl-tetradecyl-Radikalen.

7. Zusammensetzung nach einem der Ansprüche 1 bis 6, **dadurch gekennzeichnet, dass** sie ferner pro 100 % ihrer Masse 0,1 Massen-% bis 5 Massen-%, insbesondere 0,1 Massen-% bis 2 Massen-%, eines verdickenden und/oder gelierenden Polymers enthält.

8. Verfahren zur Herstellung einer Zusammensetzung nach einem der Ansprüche 1 bis 7, **dadurch gekennzeichnet, dass** es Folgendes umfasst:

- <u>wenigstens einen Schritt a)</u> des Herstellens der Fettphase (P2) durch Mischen pro 100 Massen-% der Phase (P2) von:

a) 0,2 Massen-% bis 60 Massen-%, insbesondere 2 Massen-% bis 20 Massen-%, wenigstens eines N-Acyl-aminosäurederivats der Formel (I) mit

b) 0,2 Massen-% bis 80 Massen-%, insbesondere 2 Massen-% bis 40 Massen-%, des Lösungsvermittlers;

c) 5 % bis 99,4 % eines oder mehrerer Öle und/oder eines oder mehrerer Wachse und mit

d) 0,2 Massen-% bis 80 Massen-%, insbesondere 2 Massen-% bis 40 Massen-%, des Emulgatorsystems (A); und

e) optional einem verdickenden und/oder gelierenden Polymer in der benötigten Menge, um in der Zusammensetzung 0,1 Massen-% bis 5 Massen-%, insbesondere 0,1 Massen-% bis 2 Massen-%, des verdickenden und/oder gelierenden Polymers zu erhalten,

- <u>wenigstens einen Schritt b)</u> des Emulgierens von 5 Massen-% bis 50 Massen-% der Fettphase P2 in 95 Massen-% bis 50 Massen-% der wässrigen Phase P1.

9. Verwendung der Zusammensetzung nach einem der Ansprüche 1 bis 7 als topische kosmetische Zusammensetzung durch ihre Aufbringung auf die Haut oder die Schleimhäute des menschlichen Körpers.

**Claims**

1. Composition in the form of an oil-in-water emulsion comprising, for 100% of its weight:

   (1) from 95% to 50% by weight and more particularly from 90% to 70% by weight of a cosmetically acceptable aqueous phase (P1), and
   (2) from 5% to 50% by weight and more particularly from 10% to 30% by weight of a fatty phase (P2), **characterised in that** said fatty phase (P2) comprises, for 100% of its weight:

   a) from 0.2% to 60% by weight, more particularly from 2% to 20% by weight, of at least one N-acylated amino acid derivative of formula (I):

   $$R_1\text{-C(=O)-NH-CH-(R'}_1\text{)-C(=O)-OH} \qquad (I)$$

   in which $R_1$ represents a saturated or unsaturated, linear or branched, aliphatic hydrocarbon group comprising from seven to seventeen carbon atoms, and $R'_1$ represents a hydrogen atom or a hydrocarbon group selected from methyl, benzyl, isopropyl, 1-methylpropyl or isobutyl groups, and
   b) from 0.2% to 80% by weight, more particularly from 2% to 40% by weight, of a solubilising agent comprising, for 100% of its weight:

   (i) from 5% to 100% by weight of at least one compound of formula (II):

   $$R\text{-OH} \qquad (II)$$

   in which R represents a branched alkyl group $CH(C_nH_{2n+1})(C_mH_{2m+1})\text{-CH}_2\text{-}$ in which m is an even integer between 6 and 18, n is an even integer between 4 and 18, and the sum n+m is greater than 10, and
   (ii) from 0% to 95% by weight of at least one compound of formula (III):

   $$R'O\text{-(X)p} \qquad (III)$$

   in which p represents a decimal number between 1 and 5, X represents the xylose residue and R', which may be identical to, or different from, the group R as defined above, represents a branched alkyl group $CH(C_nH_{2n+1})(C_mH_{2m+1})\text{-CH}_2\text{-}$ in which m is an even integer between 6 and 18, n is an even integer between 4 and 18, and the sum n+m is greater than 10;

   c) from 5% to 99.4% by weight, more particularly from 5% to 94% by weight, of one or more oils and/or of one or more waxes,
   d) from 0.2% to 80% by weight, more particularly from 2% to 40% by weight, of an emulsifying system (A) comprising, for 100% of its weight:

   (i) from 10% to 60% by weight of at least one of the elements of the group made up of the alkylpolyglycosides of formula (IV):

   $$R_2\text{-O-(G)}_x\text{-H} \qquad (IV)$$

   in which the group $R_2$ represents a saturated linear aliphatic group comprising from twelve to twenty two carbon atoms, G represents the residue of a reducing sugar selected from the group consisting of glucose, xylose and arabinose, and x represents a decimal number greater than or equal to 1, and less than or equal to 5, and
   (ii) from 40% to 90% by weight of at least one fatty alcohol of formula (V):

   $$R_3\text{-OH} \qquad (V)$$

in which the group $R_3$, which may be identical to, or different from, the group $R_2$ as defined above, represents a saturated linear aliphatic group comprising from 12 to 22 carbon atoms.

2. Composition according to claim 1, **characterised in that**, in formula (I), the group $R_1$-C(=O)- represents a group selected from octanoyl, decanoyl, dodecanoyl, tetradecanoyl, hexadecanoyl, octadecanoyl or octadecenoyl groups.

3. Composition according to either claim 1 or claim 2, **characterised in that**, in formula (I), the group $R'_1$ represents a hydrogen atom or a hydrocarbon group selected from methyl, isopropyl, 1-methylpropyl or isobutyl groups.

4. Composition according to claim, **characterised in that**, in formula (I), the group $R'_1$ represents a hydrogen atom or a hydrocarbon group selected from methyl, 1-methylpropyl or isobutyl groups.

5. Composition according to claim 4, wherein the N-acylated amino acid derivative of formula (I) is N-palmitoyl alanine, N-palmitoyl glycine, N-palmitoyl leucine, N-palmitoyl isoleucine or N-cocoyl alanine.

6. Composition according to any of claims 1 to 5, for which, in formulae (II) and (III), the groups R and R', which are identical or different, represent a group selected from 2-hexyldecyl, 2-hexyldodecyl, 2-octyldecyl, 2-octyldodecyl and 2-decyltetradecyl groups.

7. Composition according to any of claims 1 to 6, **characterised in that** it also comprises, for 100% of its weight, from 0.1% to 5% by weight, more particularly from 0.1 % to 2% by weight, of a thickening and/or gelling polymer.

8. Process for preparing the composition according to any of claims 1 to 7, **characterised in that** it comprises:

   - at least one step a) of preparing said fatty phase (P2) by mixing, for 100% by weight of said phase (P2):

     a) from 0.2% to 60% by weight, more particularly from 2% to 20% by weight, of at least one N-acylated amino acid derivative of formula (I), with
     b) from 0.2% to 80% by weight, more particularly from 2% to 40% by weight, of said solubilising agent;
     c) from 5% to 99.4% of one or more oils and/or of one or more waxes, and with
     d) from 0.2% to 80% by weight, more particularly from 2% to 40% by weight, of said emulsifying system (A); and
     e) optionally a thickening and/or gelling polymer, in the quantity required to obtain, in said composition, from 0.1 % to 5% by weight, more particularly from 0.1 % to 2% by weight, of said thickening and/or gelling polymer,

   - at least one step b) of emulsifying from 5% to 50% by weight of said fatty phase P2 in from 95% to 50% by weight of said aqueous phase P1.

9. Use of the composition according to any of claims 1 to 7 as a topical cosmetic composition by means of its application to the skin or the mucus membranes of the human body.

**EP 2 624 805 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1449517 A
- WO 2010023390 A
- WO 2010026325 A
- FR 2935378
- WO 9809611 A

### Littérature non-brevet citée dans la description

- **MICHEL ; IRENE ASH.** Thesaurus of Chemical Products. Chemical Publishing Co, Inc, 1986, vol. I, 211
- **DANIEL VOET ; JUDITH G. VOET.** Biochemistry. John Wyley & Sons, 1990, 250